# EUROPEAN PATENT SPECIFICATION

(11) **EP 1 951 174 B1**
(45) Date of publication and mention of the grant of the patent: **17.06.2015**
(21) Application number: 06819291.3
(22) Date of filing: 07.11.2006
(51) Int. Cl.: A61F 9/01, A61B 18/22

(54) **APPLICATOR HANDPIECE FOR LASER-INDUCED SUTURE OF THE CORNEA**
APPLIKATOR-HANDSTÜCK FÜR LASER-INDUZIERTE NÄHTE AN DER HORNHAUT
APPLICATEUR A MAIN POUR SUTURE DE LA CORNEE AU LASER

(30) Priority: 09.11.2005 IT FI20050225
(43) Date of publication of application: 06.08.2008
(73) Proprietor: CONSIGLIO NAZIONALE DELLE RICERCHE, 00185 Roma (IT)
(72) Inventor: PINI, Roberto, I-50029 Impruneta (firenze) (IT); MENABUONI, Luca, I-50033 Impruneta (firenze) (IT)
(74) Representative: Brazzini, Silvia
(86) International application number: PCT/EP2006/068170
(87) International publication number: WO 2007/054490

(56) References cited:
- WO-A-92/17243
- WO-A-95/15134
- DE-B3- 10 330 821
- US-A- 4 917 084
- US-A1- 2006 084 952

## Description

### Field of the Invention

The present invention in general regards the field of the techniques of laser-induced suture of biological tissues and its application in ophthalmologic surgery, particularly for the suture of corneal incisions in cornea transplant operations and in cataract surgery, as well as for the reparation of corneal wounds due to traumatism.

In particular, the invention refers to an applicator handpiece to be employed in contact with the corneal surface so to carry out controlled laser irradiation of the corneal incision.

### State of the art

The technique of laser-induced suture of the cornea was recently proposed in ophthalmologic surgery at an experimental level [see for example: F. Rossi, R. Pini, L. Menabuoni, R. Mencucci, U. Menchini, S. Ambrosini, G. Vannelli, "Experimental study on the healing process following laser welding of the cornea", Journal of Biomedical Optics 10, pp. 1-7 (2005)].

It is based, on the application of a biocompatible exogenous chromophore in the corneal incision to be sutured, followed by irradiation with low power diode laser (less than 100 mW). The exogenous cromophore employed is chosen so to have a strong optical absorption of the emission wavelength of the laser, to induce photochemical and photothermal activation of the stromal collagen at the incision edges. Here, the laser activation induces, on the microscopic level, the interlocking of the collagen fibrils, which makes on the macroscopic level the suture and the sealing of the corneal wound. The technique offers considerable advantages, particularly in the case of cornea transplant (perforating keratoplasty), since it permits substantially shortening surgery times, limits the use of suture material and thus favours a quicker and more efficient healing process, preventing the development of inflammatory processes typical of traditional suture with surgical thread (foreign body reaction).

Notwithstanding such above positive remarks, technical and applicative problems exist which have until now limited the diffusion of this technique in the clinical sphere. A crucial aspect for the success of the laser-induced suture is the control of local heating and therefore of the possible damage to the corneal tissue, which originates due to the coagulation of the stromal collagen for temperatures greater than 60°C, causing loss of transparency and tissue contraction. The thermal development in the corneal incision during the laser-induced suture operations essentially depends on two factors:
1) the concentration of the exogenous chromophore in the incision; this is rather easily controllable, once the exogenous chromophore solution preparation mode and the topical application mode are standardised.
2) the laser irradiating conditions, and in particular the specific power per surface unit (measured in Watt/cm²) on the corneal surface.

With reference to this second aspect, it should be observed that the irradiation is typically carried out with an optical fibre which is manually operated by the surgeon and kept at a distance of several millimetres from the corneal surface. Direct contact between the fibre and the corneal surface is in fact avoided so to not dirty the emitting face of the fibre itself, which would cause a considerable lowering of the transmitted power. In this operative mode, one never knows the exact actual distance between the fibre end and the corneal surface, and thus the specific power per surface unit of the irradiated area is not clearly determined. As is known to a person skilled in the art, the multimode optical fibres employed for these objects emit intrinsically divergent beams, with numeric aperture typically comprised between 0.2 and 0.4. This means, for example, that reducing the distance of the fibre from the surface from 2.0 to 1.5 mm, the irradiation area may decrease almost 50%, and consequently the specific laser power of the same will increase by the same percentage amount. In such variation context, uncontrolled thermal damage to the corneal tissue can occur during the laser-induced suture procedures.

Document WO-A-95/15134 discloses a laser system to deliver radiation in a defined pattern to a cornea by means of optical fibers, a handle having a terminal section in a biocompatible material with at least one face thereof transparent to laser radiation, the emitting ends of the plurality of optical fibres being incorporated within the terminal section at a predetermined distance from said face.

### Summary of the invention

The general object of the present invention is to provide a contact operated device, called "applicator handpiece" below, capable of carrying out controlled, reproducible laser irradiation, with constant specific power on the corneal surface.

A particular object of the present invention is to provide an applicator handpiece of the abovementioned type equipped with optical fibres for the transmission of the laser radiation, incorporated in a material which is transparent to the laser radiation itself so to keep the distance between the emitting ends of the fibres themselves and the corneal surface at a predetermined value.

A further object of the present invention is to provide an applicator handpiece of the abovementioned type to be employed in the technique of laser-induced suture of the cornea for applications in cornea transplant, in cataract surgery and perforating wounds.

Another particular object is to provide an applicator handpiece of the abovementioned type wherein the emitting terminal of such applicator handpiece is shaped like an arc of circumference such to be adapted to the circular shape of the corneal incision to be sutured in the case of use in cornea transplant operations.

These objects are achieved with the optical fibre applicator handpiece operable in contact with the corneal surface for inducing laser-induced suture of the cornea according to the present invention, wherein its handle has a terminal section in biocompatible material with at least one of its faces, transparent to the employed laser radiation, adapted to be held in contact with the surface to be irradiated, the emitting ends of a plurality of optical fibres being arranged within such terminal section at a predetermined distance from the transparent face, whereby the distance between said ends and said surface to be irradiated remains constant during the suture operations, and hence it is possible to control the extent of the irradiation to said surface.

In particular, in the case of use of the applicator handpiece in corneal transplants, cataract and perforating wound operations, the emitting ends of the optical fibres are aligned along an arc of circumference of diameter equal to the diameter of the corneal incision to be sutured.

### Brief description of the drawings

Further characteristics and advantages of the optical fibre applicator handpiece for carrying out laser-induced sutures of biological tissues in general and of the corneal tissue in particular according to the invention will be apparent from the following description of one of its embodiments, given as not limiting example with reference to the attached drawings wherein:
Figure 1 is a perspective view of an applicator handpiece assembly according to the present invention;
Figure 2 shows the use of the applicator handpiece on an eyeball for the laser-induced suture of the cornea in a transplant operation;
Figure 3 shows the polar vision of the cornea and the terminal of the applicator handpiece;
Figure 4 shows the base projection of the applicator handpiece;
Figure 5 is a cross section of the terminal portion of the handpiece taken along lines V-V of figure 4;
Figure 6 shows the axonometric projection of the terminal incorporating three optical fibres;
Figure 7 is a cross sectional view of the terminal of the handpiece and the cornea during the irradiation of a corneal incision which is inclined with respect to the cornea surface;
Figure 8 is a sectional view of a possible design variation of the terminal of the applicator handpiece, adapted for the suture of an incision perpendicular to the corneal surface.

### Detailed description of the invention

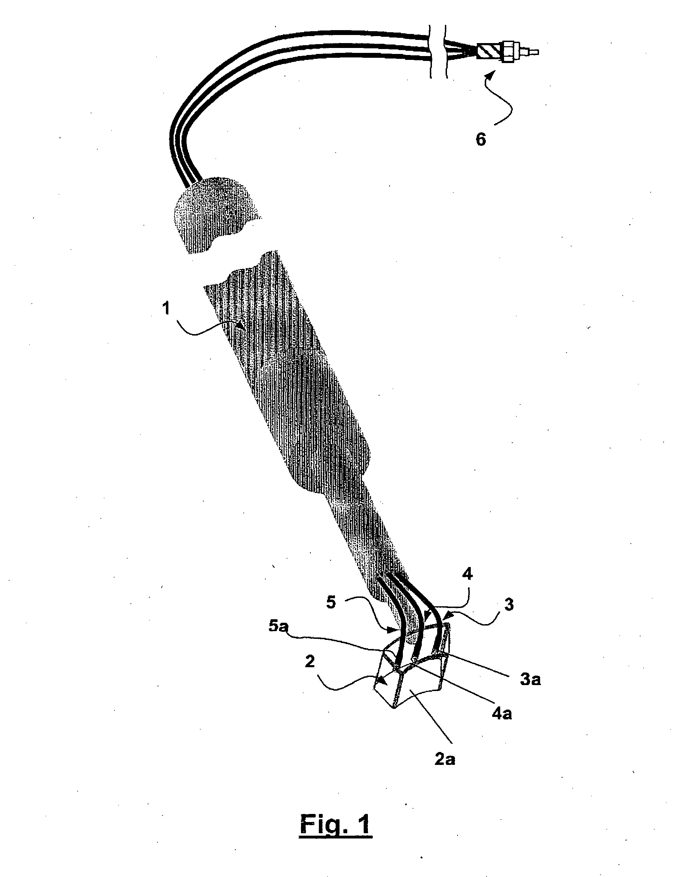

According to a preferred embodiment of the present invention, as shown in figure 1, the applicator handpiece for inducing laser-induced suture of the cornea is a device which is appropriately designed for being operated by the ophthalmologist surgeon under an operating microscope. It is therefore equipped with a handle 1, in particular of cylindrical shape, at one end of which a block terminal 2 is foreseen, made in transparent material. Three optical fibres 3, 4 and 5 have respective emitting ends 3a, 4a and 5a incorporated in the terminal 2, while the opposite ends are joined in a connector 6.

The handle 1 is tilted 45° in order to permit vision along the direction coinciding with the optical axis of the microscope, perpendicular to the corneal surface. Such tilting also permits moving the handpiece circularly along the corneal edge, avoiding the obstruction of the patient's nose.

The applicator handpiece according to the invention employs the optical fibres 3, 4 and 5 for the transmission of the laser radiation on the corneal surface. In the embodiment described here as a hot limiting example, three optical fibres were considered of 300 microns core diameter and with numeric aperture of 0.2 for the air emission. At the inlet side, the optical fibres are assembled in the connector 6 of figure 1, for example of SMA type, which can be directly connected to the diode laser not-shown (provided that the emission spot from the laser is sufficiently wide to homogeneously illuminate the inlet faces of the three fibres), or connected to an intermediate optical fibre, also not shown (for example of 600 micron diameter in the described embodiment).

The block 2 of transparent material, which makes up the terminal of the applicator handpiece, can be made in quartz, optical glass (BK7) or plastic material, such as for example polymethylmetacrylate (PMMA), which due to its stability and biocompatibility is frequently employed for making ophthalmologic aids (for example intraocular lenses). In the following description, reference will be made to a terminal composed of PMMA. Moreover, below, reference will be made to a laser-induced suture technique of the cornea which employs a diode laser with 810 nm emission (for example, the Smarty A-800 laser produced by El.En., Calenzano, FI) and the exogenous chromophore Indocyanine Green (for example the pharmacological form IC-Green produced by Akorn, Buffalo, IL, USA) as topical activator in the corneal incision; more detail on such technique is reported in the abovementioned bibliographical reference.

Considering therefore the choice of a laser with 810 nm emission, the PMMA is a perfectly suitable material for making the terminal, because it is entirely transparent to this wavelength. More in general, a block 2 can be employed wherein its face 2a turned towards the surface to be irradiated is in a material which is transparent to the employed laser radiation. It is nevertheless preferable that the block 2 is overall in optically transparent, so to not limit the surgeon's field of view.

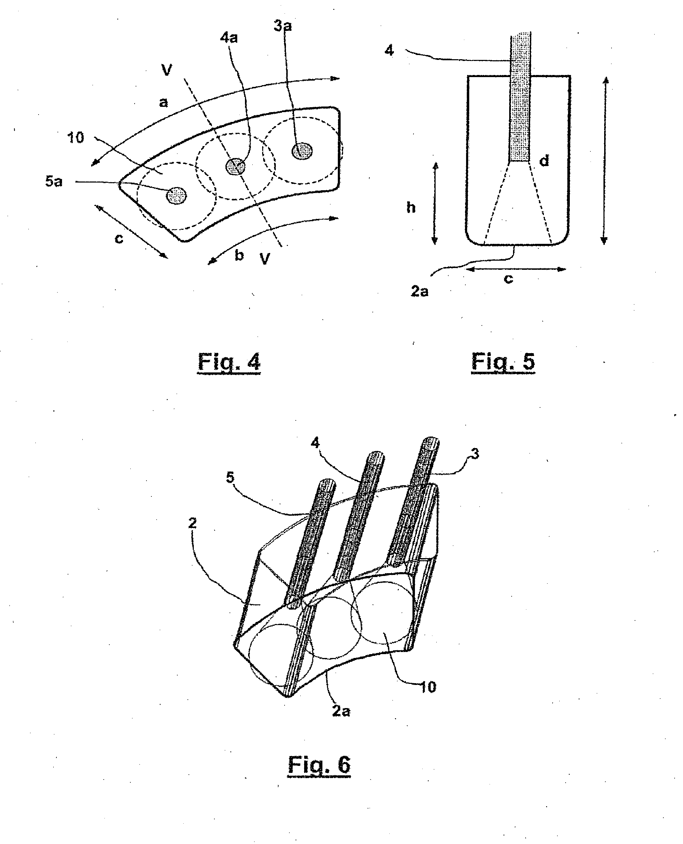

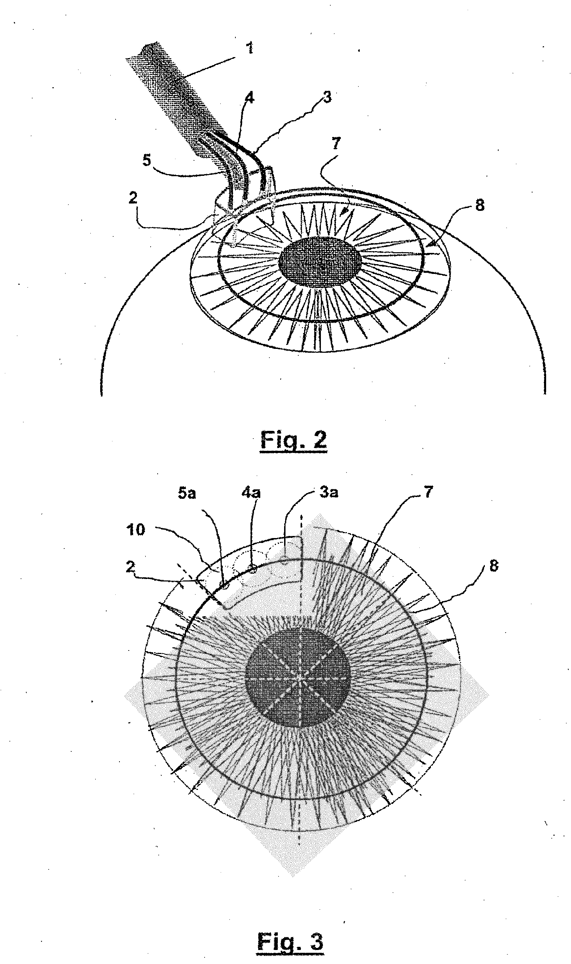

With reference to figures 2 and 3, where the donor's corneal graft is indicated with 7 and the corneal incision to be sutured with 8, the block 2 which makes up the terminal of the applicator handpiece is designed so to permit the treatment of one circular sector at a time of the corneal incision 8 to be sutured. Such circular sector is 1/8 of the entire circle which forms the transplanted graft 7. Considering a diameter of 8 mm for the latter, the terminal is made, with reference to figures 4, 5 and 6, according to the following measures: a=3.7 mm, b=2.5 mm, c=1.5 mm, d=5.0 mm. The distance h between the emitting end of the optical fibre and the base of the terminal is 1.5 mm. In these figures, the irradiation area related to each optical fibre, delimited by a circular dashed line, is indicated at 10.

As is known by a person skilled in the art, considering an optical fibre with 300 micron core and 0.2 numeric aperture, incorporated in PMMA (1.492 refraction index) with the emitting end placed at a distance h of 1.5 mm from the base of the PMMA block, it will project a light band of about 1.2 mm diameter on the base, indicated at 10 in figures 4 and 6. Therefore, three fibres of such type, assembled as described above, emit partially overlapped irradiation lobes which completely illuminate the arc corresponding to 1/8 of the circumference of the corneal incision of 8 mm diameter, as shown in figure 2.

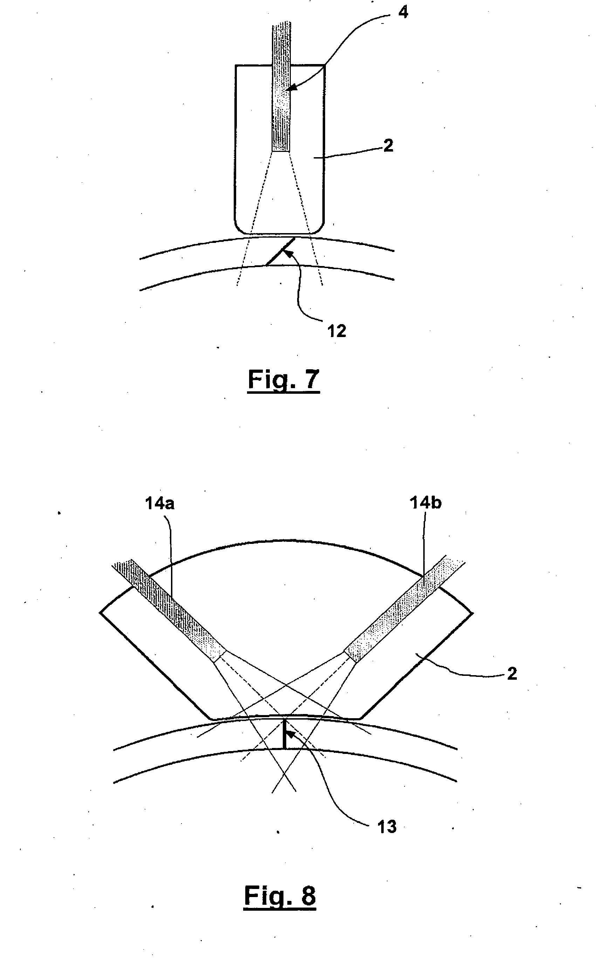

The device according to the invention is well suited for a laser-induced suture of corneal incisions which are inclined with respect to the corneal surface, as shown in figure 7. Such incisions, indicated with 12, are for example those carried out with a precalibrated scalpel in cataract operations, in order to make the corneal tunnel through which the intraocular lens is inserted. In the cornea transplant, tilted incisions are made with the recent techniques which employ femtosecond lasers for the corneal dissection (such as for example with the INTRALASE FS Neodyme:YAG laser made by Intralase Corp., Irvine, CA, USA).

A possible variation of the above described embodiment is represented in figure 8, which shows a section of the terminal 2, in which instead of three optical fibres as in the preceding case, three pairs of optical fibres are incorporated, each of which is tilted, for example 45°, with respect to the normal of the cornea. A single pair of optical fibres is shown in figure 8 and indicated with 14a and 14b. This variation has been proven advantageous in the cases wherein perpendicular irradiation of the cornea is to be avoided, such as for example when, in a corneal lesion localised in the pupillary region, one must use a metered amount of laser irradiation which is potentially dangerous for the retina, or when, in suturing an incision perpendicular to the corneal surface, one wishes to achieve the lateral irradiation of the faces of the incision, as shown in figure 8.

Further variations and/or modifications can be made to the applicator handpiece for laser-induced suture of the cornea according to the present invention without departing from the scope of the invention as set forth in the following claims.

## Claims

1. Applicator handpiece for the laser-induced suture of the corneal tissue in cornea transplant operations, wherein the transmission of the laser radiation on the surface to be irradiated is achieved by means of optical fibres (3, 4, 5), comprising a handle (1) and wherein said handle (1) has a terminal section (2) in biocompatible material with at least one face (2a) thereof transparent to the laser radiation and adapted to be held in contact with said surface, the emitting ends (3a, 4a, 5a) of a plurality of optical fibres (3, 4, 5) being incorporated within said terminal section (2) at a predetermined distance from said face(2a), so that the distance between said ends (3a, 4a, 5a) and said surface to be irradiated remains constant during the suture operations, said terminal section being formed by a block in the shape of an arc of circumference of 8 mm of diameter equal to that of the corneal incision to be sutured and having an extension in the circumferential direction equal to 1/8 of the circumference forming the incision of 8 mm of diameter, said emitting ends (3a, 4a, 5a) being aligned along said arc of circumference.

2. Applicator handpiece according to claim 1, wherein said handle (1) is inclined with respect to the perpendicular to the contact face (2a) of its terminal section (2).

3. Applicator handpiece according to claim 2, wherein said handle (1) is inclined 45° with respect to the perpendicular to the contact face (2a) of its terminal section (2).

4. Applicator handpiece according to any one of the previous claims, for the laser-induced suture of corneal incisions which are inclined with respect to the corneal surface.

5. Applicator handpiece according to any one of the previous claims, comprising three optical fibers with 300 micron core and 0.2 numeric aperture with emitting ends (3a, 4a, 5a) placed at a distance of 1.5 mm from the base of said block, said fibers emitting partially overlapped irradiation lobes which completely illuminate the arc corresponding to 1/8 of the circumference of the corneal incision of 8 mm of diameter.

6. Applicator handpiece according to any one of the previous claims, wherein said block is made of an optically transparent material.

7. Applicator handpiece according to any one of the previous claims, wherein said emitting ends (3a,4a,5a) of said optical fibers (3,4,5) are two-by-two converging along an arc of circumference of diameter equal to that of the corneal incision to be sutured.

## Patentansprüche

1. Applikator-Handstück für eine Laser-induzierte Naht an der Hornhaut bei Hornhaut-Transplantationsoperationen, wobei die Übertragung der Laserstrahlung auf die Oberfläche, die bestrahlt werden soll, mittels optischen Fasern (3, 4, 5) erzielt wird, enthaltend eine Handhabe (1), und wobei die Handhabe (1) eine Endsektion (2) aus bio-kompatiblem Material hat, wobei wenigstens eine Seitenfläche (2a) davon für die Laserstrahlung transparent ist und dazu ausgelegt ist, um in Kontakt mit der Oberfläche gehalten zu werden, wobei die emittierenden Enden (3a, 4a, 5a) einer Mehrzahl von optischen Fasern (3, 4, 5) in einem vorbestimmten Abstand von der Seitenfläche (2a) innerhalb der Endsektion (2) eingearbeitet sind, so dass der Abstand zwischen den Enden (3a, 4a, 5a) und der Oberfläche, die bestrahlt werden soll, während den Nahtoperationen konstant bleibt, wobei die Endsektion durch einen Block in der Form einer Bogenausdehnung mit 8 mm Durchmesser gebildet ist, der gleich zu jenem der Hornhaut-Inzision ist, die genäht werden soll, und eine Erstreckung in der Ausdehnungsrichtung hat, die gleich zu 1/8 der Ausdehnung ist, die die Inzision mit 8 mm Durchmesser bildet, wobei die emittierenden Enden (3a, 4a, 5a) längs der Bogenausdehnung ausgerichtet sind.

2. Applikator-Handstück nach Anspruch 1, wobei die Handhabe (1) bezüglich der Senkrechten zu der Kontaktseitenfläche (2a) ihrer Endsektion (2) geneigt ist.

3. Applikator-Handstück nach Anspruch 2, wobei die Handhabe (1) bezüglich der Senkrechten zu der Kontaktseitenfläche (2a) ihrer Endsektion (2) um 45 ° geneigt ist.

4. Applikator-Handstück nach einem der vorhergehenden Ansprüche für eine Laser-induzierte Naht von Hornhaut-Inzisionen, die bezüglich der Hornhautoberfläche geneigt sind.

5. Applikator-Handstück nach einem der vorhergehenden Ansprüche, enthaltend drei optische Fasern mit 300 Mikrometer Kern und 0,2 numerischer Apertur, mit emittierenden Enden (3a, 4a, 5a), die in einem Abstand von 1,5 mm von der Basis des Blocks platziert sind, wobei die Fasern teilweise überlappende Strahlungskeulen emittieren, die den Bogen entsprechend 1/8 der Ausdehnung der Hornhaut-Inzision mit 8 mm Durchmesser vollständig beleuchten.

6. Applikator-Handstück nach einem der vorhergehenden Ansprüche, wobei der Block aus einem optisch transparenten Material besteht.

7. Applikator-Handstück nach einem der vorhergehenden Ansprüche, wobei die emittierenden Enden (3a, 4a, 5a) der optischen Fasern (3, 4, 5) in Zweiergruppen längs einer Bogenausdehnung mit einem Durchmesser konvergieren, der gleich zu jenem der Hornhaut-Inzision ist, die genäht werden soll.

## Revendications

1. Applicateur à main pour la suture au laser du tissu cornéen lors d'opérations de transplantation de cornée, où la transmission du rayonnement laser sur la surface à irradier est réalisée au moyen de fibres optiques (3, 4, 5), comportant une poignée (1) et dans lequel ladite poignée (1) a une partie terminale (2) en un matériau biocompatible avec au moins une face (2a) transparente au rayonnement laser et adaptée pour être maintenue en contact avec ladite surface, les extrémités émissives (3a, 4a, 5a) d'une pluralité de fibres optiques (3, 4, 5) étant incorporées dans ladite partie terminale (2) à une distance prédéterminée de ladite face (2a), de façon que la distance entre lesdites extrémités (3a, 4a, 5a) et ladite surface à irradier demeure constante pendant les opérations de suture, ladite partie terminale étant formée d'un bloc en forme d'arc de cercle de 8 millimètres de diamètre égal à celui de l'incision cornéenne à suturer et ayant une extension dans la direction circulaire égale à 1/8 de la circonférence formant l'incision de 8 millimètres de diamètre, lesdites extrémités émissives (3a, 4a, 5a) étant alignées le long du dit arc de cercle.

2. Applicateur à main selon la revendication 1, où ladite la poignée (1) est inclinée par rapport à la perpendiculaire à la face de contact (2a) de sa partie terminale (2).

3. Applicateur à main selon la revendication 2, où ladite poignée (1) est inclinée à 45° par rapport à la perpendiculaire à la face de contact (2a) de sa partie terminale (2).

4. Applicateur à main selon l'une quelconque des revendications précédentes, pour la suture au laser d'incisions cornéennes qui sont inclinées par rapport à la surface cornéenne.

5. Applicateur à main selon l'une quelconque des revendications précédentes, comportant trois fibres optiques avec un noyau de 300 microns et une ouverture numérique de 0,2 avec des extrémités émissives (3a, 4a, 5a) placées à une distance de 1,5 millimètre à partir de la base du dit bloc, lesdites fibres émettant des lobes d'irradiation se recouvrant partiellement qui illuminent complètement l'arc correspondant à 1/8 de la circonférence de l'incision cornéenne de 8 millimètres de diamètre.

6. Applicateur à main selon l'une quelconque des revendications précédentes, où ledit le bloc est réalisé en un matériau optiquement transparent.

7. Applicateur à main selon l'une quelconque des revendications précédentes, où lesdites extrémités émissives (3a, 4a, 5a) des dites fibres optiques (3, 4, 5) sont convergentes deux-par-deux le long d'un arc de cercle de diamètre égal à celui de l'incision cornéenne à suturer.
